(19) Europäisches Patentamt
European Patent Office
Office européen des brevets

(11) Publication number: **0 366 033**
**A2**

(12) # EUROPEAN PATENT APPLICATION

(21) Application number: **89119592.7**

(22) Date of filing: **21.10.89**

(51) Int. Cl.[5]: **A61K 37/64 , A61K 31/00**

(30) Priority: **24.10.88 US 261206**

(43) Date of publication of application:
**02.05.90 Bulletin 90/18**

(84) Designated Contracting States:
**AT BE CH DE ES FR GB GR IT LI LU NL SE**

(71) Applicant: **E.R. SQUIBB & SONS, INC.**
**P.O.Box 4000**
**Princeton New Jersey 08543-4000(US)**

(72) Inventor: **Graney, William F.**
**10 Sturwood Way**
**Lawrenceville New Jersey 08648(US)**

(74) Representative: **Josif, Albert et al**
**Baaderstrasse 3**
**D-8000 München 5(DE)**

(54) **Method of improving post-ischemic myocardial dysfunction using an ace inhibitor alone or in combination with a thrombolytic agent and combination.**

(57) A method is provided for improving post-ischemic myocardial dysfunction such as contractile dysfunction or reperfusion injury by administering an angiotensin converting enzyme inhibitor alone or in combination with a thrombolytic agent before, during or immediately after (within 4 to 6 hours) an ischemic attack.

EP 0 366 033 A2

# METHOD OF IMPROVING POST-ISCHEMIC MYOCARDIAL DYSFUNCTION USING AN ACE INHIBITOR ALONE OR IN COMBINATION WITH A THROMBOLYTIC AGENT AND COMBINATION

The present invention relates to a method for improving post-ischemic myocardial dysfunction in mammalian species by administering an angiotensin converting enzyme (ACE) inhibitor alone or with a thrombolytic agent such as tPA which may act as scavengers of free oxygen radicals to enhance the recovery of heart function by reducing oxygen demand on the heart, and to such combination of ACE inhibitor and thrombolytic agent.

Blood flow reductions in the heart can result in dysfunction of this organ and cell death if the flow reduction is severe enough. Restoration of coronary blood flow early during a heart attack is becoming a clinical reality with the advent and improvements in thrombolytic, mechanical, and surgical interventions. While early restoration of blood flow, for example, by thrombolysis or following transient ischemia, can prevent or mitigate the degree of cell death (infarction) occurring, reperfusion can still result in some degree of cardiac dysfunction or cell death (also referred to as stunned myocardia). Thus, it would be of great clinical value to find a means to preserve reperfusion function of the heart.

In accordance with the present invention, a method is provided for treating post-ischemic myocardial dysfunction in mammalian species to improve heart function, wherein a therapeutically effective amount of an angiotensin converting enzyme inhibitor (ACE inhibitor) alone or in combination with a thrombolytic agent is systemically administered, such as orally or parenterally, or by catheter, prior to, during or soon after reperfusion, that is, within 4 to 6 hours of a myocardial infarction to mitigate the post-ischemic adverse effects of free radicals on heart function during periods of myocardial occlusion and reperfusion.

The term "reperfusion" is employed herein to refer to release of occlusion and resumption of blood flow.

In addition, in accordance with the present invention, there is provided a combination of ACE inhibitor and thrombolytic agent.

The ACE inhibitor and thrombolytic will be employed in a weight ratio to each other of within the range of from about 0.1:1 to about 10:1 and preferably from about 0.4:1 to about 2.5:1.

It has been found that the ACE inhibitor alone or in combination with a thrombolytic agent improves post-ischemic performance of the heart by improving post-ischemic contractile function when administered during both the coronary occlusion period and the reperfusion period (first 4 to 6 hours after myocardial infarction) or only during the reperfusion period. Such improvement in post-ischemic performance of the heart is evidenced by decreased contractile dysfunction, decrease in tissue necrosis, reduced myocardial workload, reduced myocardial demand for oxygen and reduced myocardial peripheral work.

The above combination may also be administered in the weeks and months following myocardial infarction to inhibit left ventricle chamber enlargement and wall thinning.

The above benefits are achieved primarily through use of a mercapto (-S-) containing ACE inhibitor alone or in conjunction with a thrombolytic agent. Administered simultaneously with the thrombolytic agent, the free radical scavenging effects of the ACE inhibitor would prevent reperfusion injury which develops when blood flow is restored to a previously ischemic area.

The angiotensin converting enzyme inhibitor which may be employed herein includes those containing a mercapto (-S-) moiety such as substituted proline derivatives, such as any of those disclosed in U. S. Patent No. 4,046,889 to Ondetti et al, with captopril, that is, 1-[(2S)-3-mercapto-2-methylpropionyl]-L-proline, being preferred, and mercaptoacyl derivatives of substituted prolines such as any of those disclosed in U. S. Patent No. 4,316,906, with zofenopril

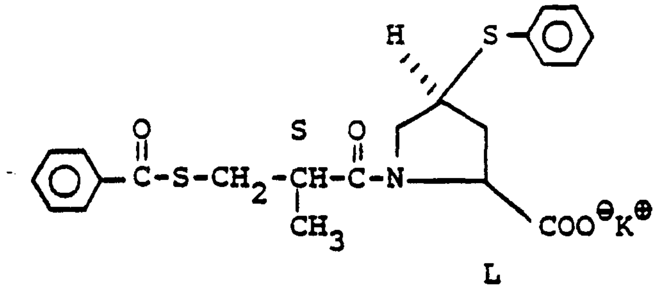

being preferred.

Other examples of ACE inhibitors that may be employed herein include rentiapril (fentiapril, Santen) disclosed in Clin. Exp. Pharmacol. Physiol. 10:131 (1983), as well as pivopril, that is

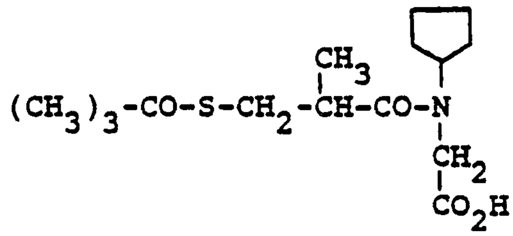

, and YS980, that is

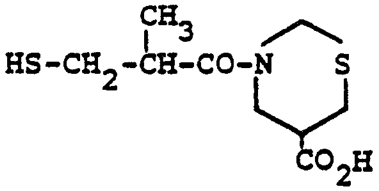

.

The disclosure of the above-mentioned patents and other references are incorporated herein by reference.

Thrombolytic agents which may be employed herein include tissue plasminogen activator (tPA), recombinant tPA, streptokinase, urokinase, prourokinase, and anisoylated plasminogen streptokinase activator complex (APSAC, Eminase, Beecham Laboratories).

In carrying out the method of the present invention, the ACE inhibitor alone or in combination with the thrombolytic agent may be administered to mammalian species, such as monkeys, dogs, cats, rats, humans, etc. during the period of coronary occlusion and/or during the period of reperfusion and/or shortly after termination of the ischemic attack, for example within 1 to 2 hours after the ischemia, within 24 to 48 hours after the ischemia and for weeks and months thereafter.

Although the ACE inhibitor or above combination may be administered systemically, such as orally or parenterally, within 1 hour after the ischemia, it is preferred that the combination be administered locally, as soon after the ischemic attack as possible, to the coronary arteries by catheter such as by arterial angiography or intracoronary injection.

With regard to dosage of the ACE inhibitor alone or in combination with a thrombolytic agent, where the drug is administered by arterial angiography, intracoronary injection or intravenously, ACE inhibitor will be employed in an amount from about 0.005 to about 30 mg/kg/treatment and preferably from about 0.01 to about 3 mg/kg/treatment will be employed. The number of treatments will depend upon the length of the ischemic attack and the progress of reperfusion to achieve normal heart function. Usually, from 1 to 5 treatments per day will be required for as long as contractile dysfunction continues.

The thrombolytic agents will be employed in amounts as indicated in the Physician's Desk Reference (PDR), 42nd Edition, 1988, Thus, Hoechst's Streptase® brand of streptokinase may be administered as follows:

TABLE I

Suggested Dilutions and Infusion Rates

| Streptase® (streptokinase) Dosage/Infusion Rate | Streptase® (streptokinase) Vial Content Needed | Total Volume of Solution (mL) | Infusion Pump Rate |
|---|---|---|---|
| I. Intracoronary Artery Administration | | | |
| A. Bolus Injection 20,000 IU | 1 vial, 250,000 IU* | 125 | Inject 10 mL |
| B. Maintenance Dose 2,000 IU/min | | | 60 mL per hour |
| *sufficient for Bolus Injection and Maintenance Dose | | | |
| II. Intravenous Administration | | | |
| A. Loading Dose 250,000 IU/30 min | a) 1 vial, 250,000 IU | 45 | 90 mL per hour for 30 min |
| | or | | |
| | b) 1 vial, 750,000 IU | 45 | 30 mL per hour for 30 min |
| B. Maintenance Dose 100,000 IU/hr | 1 vial, 750,000 IU | 45** | 6 mL per hour |

**If necessary, total volume may be increased, in increments of 45 mL, to a maximum of 500 mL with the infusion pump rate increased accordingly. The total volume of 45 mL or multiple thereof is recommended.

tPA employed herein may be Genentech's Activase®, Alteplase which, as described, in the Physician's Desk Reference (PDR), 42 Ed., 1988, Supplement A, pp. A71 and A72 is as follows.

ACTIVASE®, Alteplase, a sterile, white to off-white, lyophilized powder, is intended for intravenous administration after reconstitution with sterile water for injection, USP. The quantitative composition of the

lyophilized product is:

| 50 mg (29 million IU) Vial | 20 mg (11.6 million IU) Vial |
|---|---|
| Alteplase 50 mg (29 million IU)<br>L-Arginine 1.7 g<br>Phosphoric Acid 0.5 g<br>Polysorbate 80, less than 4 mg | Alteplase 20 mg (11.6 million IU)<br>L-Arginine 0.7 g<br>Phosphoric Acid 0.2 g<br>Polysorbate 80, less than 1.6 mg |

Phosphoric acid and/or sodium hydroxide may be used prior to lyophilization for pH adjustment.

Biological potency is determined by an in vitro clot lysis assay and is expressed in International Units as tested against the WHO standard. The specific activity of ACTIVASE®, Alteplase, is 580,000 IU/mg.

The recommended dose is 100 mg administered as 60 mg (34.8 million IU) in the first hour (of which 6 to 10 mg is administered as a bolus over the first 1-2 minutes), 20 mg (11.6 million IU) over the second hour, and 20 mg (11.6 million IU) over the third hour. For smaller patients (less than 65 kg), a dose of 1.25 mg/kg administered over 3 hours, as described above, may be used.

Abbott's Abbokinase brand of urokinase may be administered after heparin dosing of 2,500 to 10,000 units IV, in an amount by infusion into the occluded artery at a rate of 4 ml per minute (6,000 IU per minute) for periods of up to 2 hours.

Prourokinase may be administered in conventional dosages normally used in clinical practice such as a 7.5 mg bolus followed by 40.5 mg IV for 1 hour or 66.5 mg IV for 1 hour.

APSAC (Eminase) may be administered in conventional dosages normally used in clinical practice such as a single 30 unit IV dosage.

Where the ACE inhibitor or combination is to be administered by angiography, intravenously, or intracoronary injection, it will be formulated in conventional vehicles, such as distilled water, saline, Ringer's solution, or other conventional carriers.

The ACE inhibitor or combination may also be incorporated in a conventional dosage form, such as a tablet, capsule, elixir or injectable. The above dosage forms will also include the necessary carrier material, excipient, lubricant, buffer, antibacterial, bulking agent (such as mannitol), anti-oxidants (ascorbic acid of sodium bisulfite) or the like. Oral dosage forms are preferred, although parenteral forms are quite satisfactory as well.

With regard to such systemic formulations, single or divided doses of from about 5 to about 2500 mg, preferably from about 10 to 2000 mg/one t four times daily, may be administered in systemic dosage forms as described above for a period sufficient to restore normal heart function.

The dose administered must be carefully adjusted according to age, weight and condition of the patient, as well as the route of administration, dosage form and regimen and the desired result.

Thus, for oral administration, a satisfactory result may be obtained employing the ACE inhibitor in an amount within the range of from about 0.01 mg/kg to about 100 mg/kg and preferably from about 0.1 mg/kg to about 25 mg/kg alone or in combination with the thrombolytic agent in an amount within the range of from about 0.01 mg/kg to about 100 mg/kg and preferably from about 0.1 mg/kg to about 25 mg/kg with the ACE inhibitor and thrombolytic agent being employed together in the same oral dosage form or in separate oral dosage forms taken at the same time.

A preferred oral dosage form, such as tablets or capsules, will contain the ACE inhibitor in an amount of from about 0.1 to about 500 mg, preferably from about 125 to about 200 mg, and more preferably from about 25 to about 150 mg, alone or with the thrombolytic agent in an amount of from about 1 to about 350 mg, preferably from about 2 to about 200 mg, and more preferably from about 30 to about 150 mg.

For parenteral administration, the ACE inhibitor will be employed in an amount within the range of from about 0.005 mg/kg to about 10 mg/kg and preferably from about 0.01 mg/kg to about 1 mg/kg, alone or with the thrombolytic agent in an amount within the range of from about 0.005 mg/kg to about 20 mg/kg and preferbly from about 0.01 mg/kg to about 2 mg/kg.

The composition described above may be administered in the dosage forms as described above in single or divided doses of one to four times daily. It may be advisable to start a patient on a low dose combination and work up gradually to a high dose combination.

Tablets of various sizes can be prepared, e.g., of about 50 to 700 mg in total weight, containing one or both of the active substances in the ranges described above, with the remainder being a physiologically acceptable carrier of other materials according to accepted pharmaceutical practice. These tablets can, of course, be scored to provide for fractional doses. Gelatin capsules can be similarly formulated.

Liquid formulations can also be prepared by dissolving or suspending one or the combination of active substances in a conventional liquid vehicle acceptable for pharmaceutical administration so as to provide the desired dosage in one to four teaspoonsful.

Such dosage forms can be administered to the patient on a regimen of one to four doses per day.

The following Examples represent preferred embodiments of the present invention.

## Example 1

An injectable solution for use in administering ACE inhibitor by intracoronary injection by arterial angiography or intravenously is produced as follows:

| | |
|---|---|
| Captopril | 500 mg |
| Methyl paraben | 5 mg |
| Propyl paraben | 1 mg |
| Sodium chloride | 25 g |
| Water for injection qs. | 5 l. |

The captopril, preservatives and sodium chloride are dissolved in 3 liters of water for injection and then the volume is brought up to 5 liters. The solution is filtered through a sterile filter and aseptically filled into presterilized vials which are then closed with presterilized rubber closures. Each vial contains 5 ml of solution in a concentration of 100 mg of active ingredient per ml of solution for injection.

## Example 2

Tablets for use in improving post-ischemic contractile dysfunction are prepared as described in Example 1 except that zofenopril is used in place of captopril.

## Example 3

An injectable for use in improving post-ischemic contractile dysfunction is prepared as described in Example 1 except that 1-(3-mercapto-2-D-methylpropanoyl)-L-proline is employed in place of captopril.

## Example 4

A captopril formulation suitable for oral administration in improving post-ischemic contractile function is set out below.

1000 tablets each containing 100 mg of captopril were produced from the following ingredients.

| | |
|---|---|
| Captopril | 100 g |
| Corn starch | 50 g |
| Gelatin | 7.5 g |
| Avicel (microcrystalline cellulose) | 25 g |
| Magnesium stearate | 2.5 g |

The captopril and corn starch are admixed with an aqueous solution of the gelatin. The mixture is dried and ground to a fine powder. The Avicel and then the magnesium stearate are admixed with the granulation.

This is then compressed in a tablet to form 1000 tablets each containing 100 mg of active ingredient which is used for improving post-ischemic contractile function.

Example 5

The injectable solution for use in administering an ACE inhibitor, may be employed in conjunction with conventional dosage forms of tPA for reducing myocardial infarct size and improving post-ischemic contractile dysfunction.

Example 6

An injectable for use in improving post-ischemic contractile dysfunction is prepared as described in Example 5 except that the ACE inhibitor zofenopril is employed in place of captopril.

Example 7

An injectable solution for use in administering ACE inhibitor and thrombolytic agent by intracoronary injection, by arterial angiography or intravenously containing rentiapril as the ACE inhibitor and streptokinase (conventional dosage) as the thrombolytic agent is prepared as described in Example 5.

Example 8

An injectable for use in improving post-ischemic contractile dysfunction is prepared as described in Example 5 except that the ACE inhibitor employed is pivopril and the thrombolytic agent employed is urokinase.

**Claims**

1. A mercapto containing angiotensin-converting enzyme inhibitor alone or in combination with a thrombolytic agent for use in improving post-ischemic myocardial dysfunction in a mammalian species, by reducing or eliminating reperfusion injury.

2. The angiotensin converting enzyme inhibitor or the combination of the angiotensin-converting enzyme inhibitor and thrombolytic agent for use as defined in claim 1 by arterial angiography or by intracoronary injection, intravenously or orally.

3. The angiotensin converting enzyme inhibitor or the combination of the angiotensin-converting enzyme inhibitor and thrombolytic agent for use as defined in claim 1 prior to, during or after reperfusion.

4. The angiotensin converting enzyme inhibitor or the combination of the angiotensin-converting enzyme inhibitor and thrombolytic agent for use as defined in claim 1 during coronary occlusion and reperfusion.

5. The angiotensin converting enzyme inhibitor or the combination of the angiotensin-converting enzyme inhibitor and thrombolytic agent for use as defined in claim 1 during reperfusion.

6. The angiotensin converting enzyme inhibitor for use as defined in claim 1 which inhibitor is a mercapto containing substituted proline derivative.

7. The angiotensin converting enzyme inhibitor for use as defined in claim 1, which inhibitor is captopril, zofenopril, fentiapril or

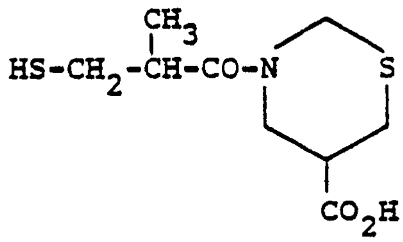

8. The thrombolytic agent for use as defined in claim 1, which is tissue plasminogen activator (tPA) recombinant tissue plasminogen activator, streptokinase, urokinase or prourokinase.

9. The angiotensin converting enzyme inhibitor for use in combination with the thrombolytic agent according to claim 8, wherein the inhibitor is captopril or zofenopril.

10. The angiotensin converting enzyme inhibitor for use as defined in claim 9 wherein the thrombolytic agent is tissue plasminogen activator or recombinant tissue plasminogen activator.

11. A pharmaceutical combination useful for improving post-ischemic myocardial dysfunction comprising an angiotensin converting enzyme inhibitor and a thrombolytic agent.

12. The combination as defined in claim 11 wherein the angiotensin converting enzyme inhibitor and thrombolytic agent are in a weight ratio to each other within the range of from about 0.1:1 to about 10:1.

13. The combination as defined in claim 11 wherein the angiotensin converting enzyme inhibitor is a mercapto containing substituted proline and the thrombolytic agent is tissue plasminogen activator, recombinant tissue plasminogen activator, streptokinase, urokinase or prourokinase or anisoylated plasminogen streptokinase activator complex (APSAC).

14. The combination as defined in claim 13 wherein the angiotensin converting enzyme inhibitor is captopril or zofenopril.

15. A pharmaceutical composition useful for improving post-ischemic myocardial dysfunction in a mammalian species characterized by having incorporated therein an angiotensin converting enzyme inhibitor and optionally a thrombolytic agent.

16. A process for preparing a composition useful for improving post-ischemic myocardial dysfunction in a mammalian species characterized by mixing an effective amount of an angiotensin converting enzyme inhibitor and optionally an effective amount of a thrombolytic agent with a conventional vehicle used for systemic application.

17. A process for preparing a pharmaceutical composition useful for improving post-ischemic myocardial dysfunction characterized by mixing effective amounts of an angiotensin converting enzyme inhibitor and a thrombolytic agent.